# EUROPEAN PATENT APPLICATION

(11) **EP 4 353 728 A1**
(43) Date of publication of application: **17.04.2024**
(21) Application number: 22820346.9
(22) Date of filing: 10.06.2022
(51) Int. Cl.: C07D 495/04, C07B 61/00

(54) **HYDROXY THIENOIMIDAZOLE DERIVATIVE, VINYL SULFIDE DERIVATIVE, N-BUTYLIDENE SULFIDE DERIVATIVE, AND PRODUCTION METHOD FOR SATURATED STRAIGHT-CHAIN HYDROCARBON-SUBSTITUTED THIENOIMIDAZOLE DERIVATIVE**

(30) Priority: 11.06.2021 JP 2021098332
(71) Applicant: Tokuyama Corporation, Shunan-shi, Yamaguchi 745-8648 (JP)
(72) Inventor: SEKI Masahiko, Shunan-shi, Yamaguchi 745-8648 (JP); MASHIMA Kazushi, Suita-shi, Osaka 565-0871 (JP); TSURUGI Hayato, Suita-shi, Osaka 565-0871 (JP); KATO Daiki, Suita-shi, Osaka 565-0871 (JP)
(74) Representative: Hoffmann Eitle
(86) International application number: PCT/JP2022/023481
(87) International publication number: WO 2022/260168

(57) **Abstract**

It is an object to provide a high-yield production method of a hydroxythienoimidazole derivative, a vinyl sulfide derivative, an n-butylidene sulfide derivative, and a thienoimidazole derivative substituted with a straight-chain saturated hydrocarbon, and, in order to achieve the object, a production method according to one embodiment includes a step of mixing a thiolactone derivative represented by formula (I) with a Grignard reagent represented by formula (1) or (2) in the presence of a copper salt to obtain a hydroxythienoimidazole derivative represented by formula (II) or (IV).

## Description

### FIELD OF THE INVENTION

The present invention relates to a method of producing a hydroxythienoimidazole derivative, a vinyl sulfide derivative, an n-butylidene sulfide derivative and a thienoimidazole derivative substituted with a straight-chain saturated hydrocarbon.

### BACKGROUND ART

Biotin, which is represented by the following formula, is considered to be a type of vitamin useful as animal feed and a medicament.

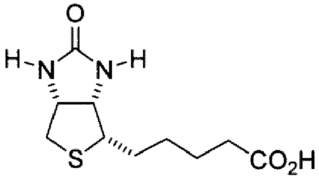

Non-Patent Document 1 discloses a method of synthesizing biotin by using a Grignard reagent, ClMg(CH₂)₃OMe. Specifically, as shown below, this document discloses a method of obtaining biotin as follows. First, thiolactone compound (A) is subjected to an addition reaction with the Grignard reagent. Then, the resulting product is hydrolyzed to obtain intermediate (B), which is dehydrated to obtain compound (C). Subsequently, compound (C) is hydrogenated to obtain compound (D). Lastly, compound (D) is subjected to coupling with a malonic ester and deprotection to obtain biotin. In the following formulas, "Bn" represents a benzyl group and "Me" represents a methyl group.

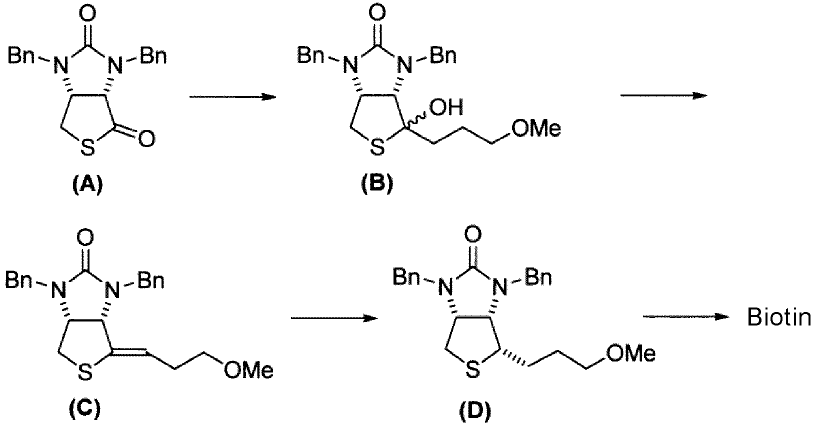

### PRIOR ART DOCUMENT

### NON-PATENT DOCUMENT

Non-Patent Document 1: M. Gerecke, J. -P. Zimmermann, W. Aschwanden, "116. Versuche zur Biotinsynthese. Herstellung von (3aS, 6aR)-1,3-Dibenzyl-tetrahydro-4H-thieno[3,4-d]imidazol-2,4(1H)-dion" Helv. Chim. Acta. 1970, 53, 991-999.

### SUMMARY OF THE INVENTION

It is an object of the present invention to provide a high-yield production method of a hydroxythienoimidazole derivative, a vinyl sulfide derivative, an n-butylidene sulfide derivative, and a thienoimidazole derivative substituted with a straight-chain saturated hydrocarbon.

According to one embodiment, there is provided a method of producing a hydroxythienoimidazole derivative represented by the following formula (II). This production method includes a step of mixing a thiolactone derivative represented by the following formula (I), a Grignard reagent represented by the following formula (1) and a copper salt to obtain a hydroxythienoimidazole derivative represented by the following formula (II).

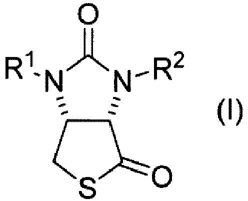

In formula (I), R¹ and R² are each independently an alkyl group, an alkyl group having a substituent, an aryl group, or an aryl group having a substituent.

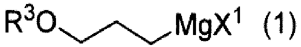

In formula (1), R³ is an alkyl group, an alkyl group having a substituent, an aryl group, or an aryl group having a substituent, and X¹ is a halogen atom.

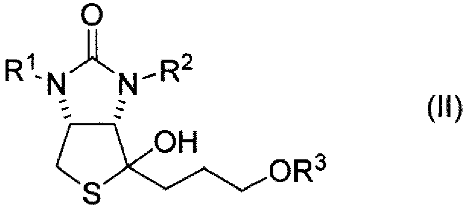

In formula (II), R¹ and R² are the same as defined in formula (I), and R³ is the same as defined in formula (1).

According to another embodiment, there is provided a method of producing a hydroxythienoimidazole derivative represented by the following formula (IV). This production method includes a step of mixing a thiolactone derivative represented by the above-described formula (I), a Grignard reagent represented by the following formula (2) and a copper salt to obtain a hydroxythienoimidazole derivative represented by the following formula (IV).

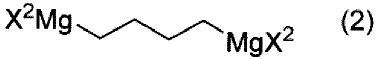

In formula (2), X² is each independently a halogen atom.

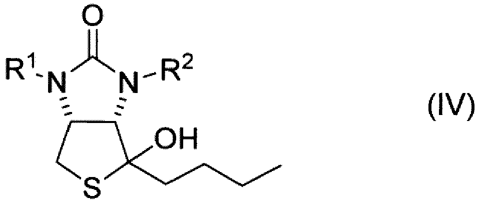

In formula (IV), R¹ and R² are the same as defined in formula (I).

According to yet another embodiment, there is provided a method of producing a vinyl sulfide derivative represented by the following formula (III). This production method includes a step of obtaining a hydroxythienoimidazole derivative represented by the above-described formula (II) by the method according to the above-described embodiment, and a step of dehydrating the resulting hydroxythienoimidazole derivative to obtain a vinyl sulfide derivative represented by the following formula (III).

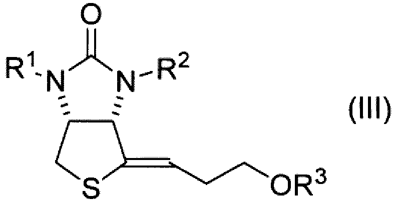

In formula (III), R¹ and R² are the same as defined in formula (I), and R³ is the same as defined in formula (1).

According to yet another embodiment, there is provided a method of producing a thienoimidazole derivative substituted with a straight-chain saturated hydrocarbon represented by the following formula (VI). This production method includes a step of obtaining a vinyl sulfide derivative represented by the above-described formula (III) by the method according to the above-described embodiment, and a step of contacting the resulting vinyl sulfide derivative with hydrogen in the presence of a catalyst, to obtain a thienoimidazole derivative substituted with a straight-chain saturated hydrocarbon represented by the following formula (VI).

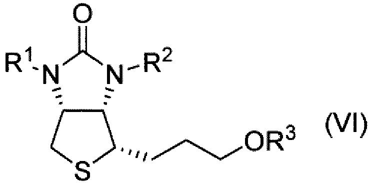

In formula (IV), R¹ and R² are the same as defined in formula (I), and R³ is the same as defined in formula (1).

According to yet another embodiment, there is provided a method of producing an n-butylidene sulfide derivative represented by the following formula (V). This production method includes a step of obtaining a hydroxythienoimidazole derivative represented by the above-described formula (IV) by the method according to the above-described embodiment, and a step of dehydrating the resulting hydroxythienoimidazole derivative to obtain an n-butylidene sulfide derivative represented by the following formula (V).

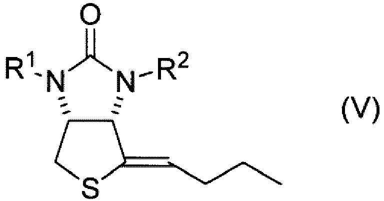

In formula (V), R¹ and R² are the same as defined in formula (I).

According to the present invention, there is provided a high-yield production method of a hydroxythienoimidazole derivative, a vinyl sulfide derivative, an n-butylidene sulfide derivative, and a thienoimidazole derivative substituted with a straight-chain saturated hydrocarbon.

### DETAILED DESCRIPTION OF THE INVENTION

A production method according to one embodiment includes a step of mixing a thiolactone derivative represented by formula (I) with a Grignard reagent represented by formula (1) or (2) in the presence of a copper salt to obtain a hydroxythienoimidazole derivative represented by formula (II) or (IV). The hydroxythienoimidazole derivative may be used as an intermediate for the synthesis of, for example, biotin and a biotin derivative.

This production method makes it possible to obtain the hydroxythienoimidazole derivative in high yield. Copper (Cu) has a high affinity for sulfur (S). Thus, Cu in the copper salt tends to coordinate to the S atom in the thiolactone derivative, and consequently activates the S atom site of the thiolactone derivative. This allows the Grignard reagent to readily react with a carbon atom of the thiolactone derivative, wherein the carbon atom is adjacent to the S atom and forms a carbonyl group. This production method is advantageous compared with, for example, a conventional method in which no copper salt is added. This production method makes it possible to use a minimum necessary amount of the Grignard reagent to complete the reaction at a low temperature and in a short time. Furthermore, this production method makes it possible to obtain a hydroxythienoimidazole derivative in high yield without using an expensive catalyst such as a palladium catalyst. Hence, biotin can be produced efficiently at low cost.

Hereinafter, the production method according to an embodiment is described in detail.

### <Thiolactone Derivative Represented by Formula (I)>

The thiolactone derivative is represented by the following formula (I). The thiolactone derivative may be used as an intermediate for the biotin synthesis as described above.

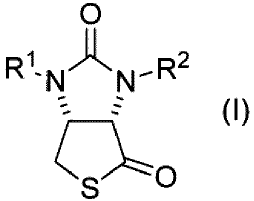

In formula (I), R¹ and R² are each independently an alkyl group, an alkyl group having a substituent, an aryl group, or an aryl group having a substituent. R¹ and R² may be a functional group identical to each other or may be a functional group different from each other. R¹ and R² are, each independently, preferably an alkyl group having a substituent, more preferably an alkyl group having a phenyl group, and even more preferably a benzyl group.

The alkyl group represented by R¹ or R² may be a straight chain or a branched chain. The number of carbon atoms of the alkyl group represented by R¹ or R² is, for example, 1 to 20, preferably 1 to 10, more preferably 1 to 8, even more preferably 1 to 6, even more preferably 1 to 4, even more preferably 1 to 3, even more preferably 1 or 2, and even more preferably 1.

The alkyl group represented by R¹ or R² may have a substituent. Examples of the substituent that the alkyl group represented by R¹ or R² may have include an aryl group having 3 to 20 (preferably 6 to 20, more preferably 6 to 14, and even more preferably 6 to 10) carbon atoms, an alkoxy group having 1 to 6 (preferably 1 to 4, more preferably 1 to 3, and even more preferably 1 or 2) carbon atoms, and a halogen atom. The aryl group may be a cyclic aromatic hydrocarbon group that is monocyclic, bicyclic or tricyclic. The aryl group is preferably a monocyclic one having 3 to 8 membered ring, and more preferably a phenyl group. The alkoxy group may be a straight chain or a branched chain. The halogen atom may be selected from a fluorine atom, a chlorine atom, a bromine atom and an iodine atom. The substituent that the alkyl group represented by R¹ or R² may have is preferably an aryl group having 3 to 8 carbon atoms, and more preferably a phenyl group. In cases where the alkyl group represented by R¹ or R² has a substituent, the alkyl group represented by R¹ or R² has, for example, 1 to 5 substituents, preferably 1 to 3 substituents, more preferably 1 or 2 substituents, and even more preferably 1 substituent.

The aryl group represented by R¹ or R² may be a cyclic aromatic hydrocarbon group that is monocyclic, bicyclic or tricyclic. The aryl group represented by R¹ or R² is preferably a monocyclic one having 3 to 8 membered ring. The number of carbon atoms of the aryl group represented by R¹ or R² is, for example, 3 to 30, preferably 3 to 20, more preferably 6 to 20, even more preferably 6 to 14, and still more preferably 6 to 10. The aryl group represented by R¹ or R² is preferably a phenyl group.

The aryl group represented by R¹ or R² may have a substituent. Examples of the substituent that the aryl group represented by R¹ or R² may have include an alkyl group having 1 to 6 (preferably 1 to 4, more preferably 1 to 3, and even more preferably 1 or 2) carbon atoms, an alkoxy group having 1 to 6 (preferably 1 to 4, more preferably 1 to 3, and even more preferably 1 or 2) carbon atoms, a carboxyl group, and a halogen atom. The alkyl group and the alkoxy group may be a straight chain or a branched chain. The halogen atom may be selected from a fluorine atom, a chlorine atom, a bromine atom and an iodine atom. In cases where the aryl group represented by R¹ or R² has a substituent, the aryl group represented by R¹ or R² has, for example, 1 to 5 substituents, preferably 1 to 3 substituents, more preferably 1 or 2 substituents, and even more preferably 1 substituent.

### <First Grignard Reagent>

The first Grignard reagent is represented by the following formula (1).

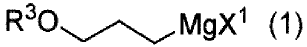

In formula (1), R³ is an alkyl group, an alkyl group having a substituent, an aryl group, or an aryl group having a substituent. The above descriptions of the alkyl group, the alkyl group having a substituent, the aryl group and the aryl group having a substituent that are represented by R¹ or R² also applies to the alkyl group, the alkyl group having a substituent, the aryl group and the aryl group having a substituent that are represented by R³. Examples of the alkyl group, the alkyl group having a substituent, the aryl group and the aryl group having a substituent that are represented by R³ include the same as those listed for R¹ and R². R³ is preferably an alkyl group having 1 to 6 carbon atoms, and more preferably a methyl group or an ethyl group.

X¹ is a halogen atom. The halogen atom may be selected from a fluorine atom, a chlorine atom, a bromine atom and an iodine atom. Preferably, the halogen atom is a chlorine atom or a bromine atom.

The first Grignard reagent is obtained, for example, by contacting an organohalogen derivative represented by the following formula (1a) with magnesium.

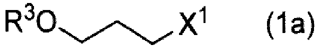

In formula (1a), R³ and X¹ are the same as defined in formula (1).

The magnesium used is in the form of a simple substance. The magnesium used may be in the form of a powder or a strip.

A contact temperature at which the organohalogen derivative represented by formula (1a) is contacted with magnesium is, for example, 40 to 150°C, and preferably 60 to 100°C. A contact time for which the organohalogen derivative represented by formula (1a) is contacted with magnesium is, for example, 10 minutes to 10 hours, and preferably 1 hour to 5 hours.

The amount of the organohalogen derivative represented by formula (1a) is, for example, 0.1 to 2 mol, preferably 0.5 to 1.5 mol, relative to 1 mol of magnesium.

The organohalogen derivative represented by formula (1a) is contacted with magnesium preferably in the presence of a magnesium activator. As the magnesium activator, at least one magnesium activator selected from the group consisting of 1,2-dibromoethane, bromine, iodine and trimethylsilyl chloride can be used. The amount of the magnesium activator is, for example, 0.01 to 1.5 mol, and preferably 0.2 to 0.8 mol, relative to 1 mol of magnesium.

The organohalogen derivative represented by formula (1a) is contacted with magnesium preferably in the presence of a first reaction solvent. As the first reaction solvent, at least one solvent selected from the group consisting of acetonitrile, propionitrile, tetrahydrofuran (THF), 2-methyl-tetrahydrofuran, 1,4-dioxane, tert-butyl methyl ether, diisopropyl ether, dimethyloxyethane, diglyme, acetone, methyl ethyl ketone, diethyl ketone, methyl acetate, ethyl acetate, butyl acetate, methylene chloride, chloroform, carbon tetrachloride, 1,2-dichloroethane, chlorobenzene, toluene, xylene, hexane and heptane can be used.

The amount of the first reaction solvent used in the production of the first Grignard reagent is, for example, 0.001 to 10 mL, and preferably 0.01 to 1 mL, relative to 1 mg of magnesium.

In cases where the first reaction solvent is used, the first Grignard reagent is prepared preferably by the following method. First, the organohalogen derivative represented by formula (1a) is mixed with one half of the first reaction solvent to prepare a solution of the organohalogen derivative. Then, the magnesium, the magnesium activator and the other half of the first reaction solvent are mixed to obtain a first mixture. The solution of the organohalogen derivative is added dropwise to the first mixture to obtain a second mixture. The second mixture is heated to the above-described contact temperature, and subsequently stirred over the above-described contact time to obtain the first Grignard reagent.

### <Copper Salt>

The valence number of a copper atom contained in the copper salt is preferably one or two, and more preferably one. A copper salt containing mono-valent copper atom has an excellent catalytic action. As the copper salt, at least one copper salt selected from copper (I) chloride (CuCl), copper (II) chloride (CuCl₂), copper (I) bromide (CuBr), copper (II) bromide (CuBr₂), copper (I) cyanide (CuCN), 3-copper (I) methylsalicylate, mesitylene copper (I) (MesCu), isopropoxy copper (I) (ⁱPrOCu), copper iodide (I) (CuI), copper (II) iodide (CuI₂), copper (I) acetate (CuOAc), copper (II) acetate (Cu(OAc)₂), copper (II) sulfate (CuSO₄), copper (I) oxide (Cu₂O), copper (II) oxide (CuO), copper (I) pivalate (CuOPiv), copper (II) pivalate (Cu(OPiv)₂), a sulfur-containing copper salt and the like can be used. The sulfur-containing copper saltused is preferably copper (I) thiophene-2-carboxylate. Of the copper salts containing mono-valent copper atom, CuCl, CuI or CuBr is particularly preferably. CuCl, CuI and CuBr each have a particularly excellent catalytic action.

### <Method of Producing Hydroxythienoimidazole Derivative Represented by Formula (II)>

The method of producing the hydroxythienoimidazole derivative represented by formula (II) includes a step of mixing the thiolactone derivative represented by formula (I), the first Grignard reagent and the copper salt.

When the thiolactone derivative represented by formula (I) is mixed with the Grignard reagent represented by formula (1) in the presence of the copper salt, an addition reaction proceeds to generate an intermediate represented by the following formula (Ia). Then, it is believed that hydrolysis of this intermediate gives a hydroxythienoimidazole derivative represented by formula (II). This hydrolysis reaction can be performed by treating the intermediate with water or an acid. As the acid, at least one selected from hydrochloric acid, sulfuric acid, phosphoric acid, methanesulfonic acid, para-toluenesulfonic acid, formic acid, acetic acid, propionic acid, ammonium chloride and silica gel can be used.

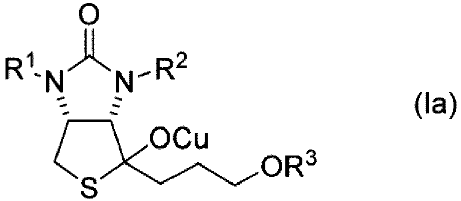

The amount of the copper salt used is preferably 0.05 to 1 mol, relative to 1 mol of the first Grignard reagent. The amount of the copper salt used is more preferably 0.5 to 0.8 mol, and still more preferably 0.6 to 0.72 mol, relative to 1 mol of the first Grignard reagent.

The amount of the copper salt used is usually 0.1 to 10 mol, preferably 0.5 to 5 mol, and more preferably 0.5 to 2 mol, relative to 1 mol of the thiolactone derivative.

The amount of the first Grignard reagent used is usually 0.5 to 10 mol, preferably 1.0 to 5 mol, and more preferably 1.0 to 2.0 mol, relative to 1 mol of the thiolactone derivative.

The thiolactone derivative, the first Grignard reagent and the copper salt may be mixed with one another in the presence of a second reaction solvent. As the second reaction solvent, at least one solvent selected from tetrahydrofuran (THF), 2-methyl-tetrahydrofuran, 1,4-dioxane, tert-butyl methyl ether, cyclopentyl methyl ether, dimethoxyethane, diglyme, methylene chloride, toluene, xylene, hexane, heptane and the like can be used. As the second reaction solvent, a single solvent may be used alone or a mixed solvent containing a combination of two or more solvents may be used. The second reaction solvent is preferably THF, toluene or a mixed solvent of these.

The amount of the second reaction solvent used is, for example, 1 to 100 mL, and preferably 2 to 50 mL, relative to 1 g of the thiolactone derivative.

When the thiolactone derivative, the first Grignard reagent and the copper salt are mixed with one another, the mixing temperature is, for example, within the range of -40 to 100°C. The mixing temperature is within the range of preferably -20 to 40°C, and more preferably -10 to 20°C. When the mixing temperature falls within the above temperature range, the yield of the hydroxythienoimidazole derivative tends to increase.

When the thiolactone derivative, the first Grignard reagent and the copper salt are mixed with one another, the mixing time is usually 0.5 to 72 hours, and preferably 1 to 48 hours.

Preferably, the thiolactone derivative, the first Grignard reagent and the copper salt are mixed by the following method.

First, the first Grignard reagent and the copper salt are mixed to obtain an organocopper reagent. The first Grignard reagent may be used in the form of a first Grignard reagent solution prepared by dissolving the first Grignard reagent in the first or second reaction solvent. The copper salt may be used in the form of a copper salt solution prepared by dissolving the copper salt in the second reaction solvent. The mixing temperature for the first Grignard reagent and the copper salt may be any temperature within the above-described mixing temperature range for the thiolactone derivative, the first Grignard reagent and the copper salt. The mixing time for the first Grignard reagent and the copper salt is set at, for example, 1 minute to 1 hour.

In the organocopper reagent, the first Grignard reagent and the copper salt are believed to form a copper complex represented by the following formula (3).

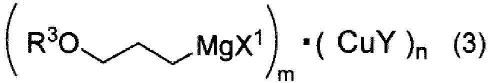

In formula (3), R³ and X¹ are the same as defined in formula (1). Y represents an anion of the copper salt. m and n each represent an integer of 1 to 3.

Next, this organocopper reagent is contacted with the thiolactone derivative represented by formula (I). The thiolactone derivative represented by formula (I) may be used in the form of a thiolactone derivative solution prepared by dissolving the thiolactone derivative in the second reaction solvent. The contact temperature for the organocopper reagent and the thiolactone derivative may be any temperature within the above-described mixing temperature range for the thiolactone derivative, the first Grignard reagent and the copper salt. The contact time for the organocopper reagent and the thiolactone derivative may be any time within the above-described mixing time range for the thiolactone derivative, the first Grignard reagent and the copper salt.

### <Hydroxythienoimidazole Derivative Represented by Formula (II)>

The hydroxythienoimidazole derivative is represented by the following formula (II). The hydroxythienoimidazole derivative may be used as an intermediate for the biotin synthesis as described above.

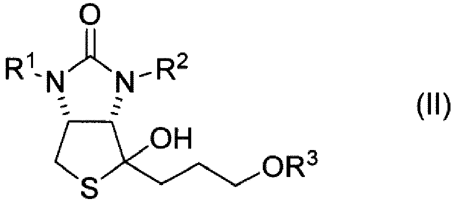

In formula (II), R¹ and R² are the same as defined in formula (I). R³ is the same as defined in formula (1).

The hydroxythienoimidazole derivative is converted into biotin by a known method.

For example, first, the hydroxythienoimidazole derivative represented by formula (II) is dehydrated to obtain a vinyl sulfide derivative represented by the following formula (III).

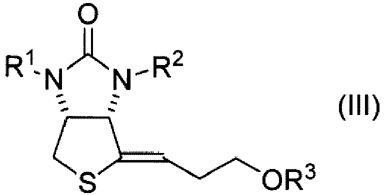

In formula (III), R¹ and R² are the same as defined in formula (I). R³ is the same as defined in formula (1).

Examples of the method of dehydrating the hydroxythienoimidazole derivative include acid treatment and heat treatment. Acid treatment includes, for example, contacting the hydroxythienoimidazole derivative represented by formula (II) with an acid catalyst. Examples of the acid catalyst include sulfuric acid, hydrochloric acid and a mixture thereof. The temperature for heat treatment is, for example, -20 to 120°C, and preferably 0 to 70°C. Acid treatment and heat treatment may be used in combination.

Next, the vinyl sulfide derivative represented by formula (III) is hydrogenated in the presence of, for example, a Pd catalyst to obtain a compound represented by the following formula (VI). The resulting compound represented by the following formula (VI) is reacted with hydrogen bromide to obtain a compound represented by the following formula (VIII). Then, the resulting compound is reacted with CH₂(COOEt)₂ to obtain a compound represented by the following formula (XI). The compound obtained is debenzylated and subsequently treated with, for example, hydrogen bromide to yield biotin. In the following formulas, "Et" represents an ethyl group.

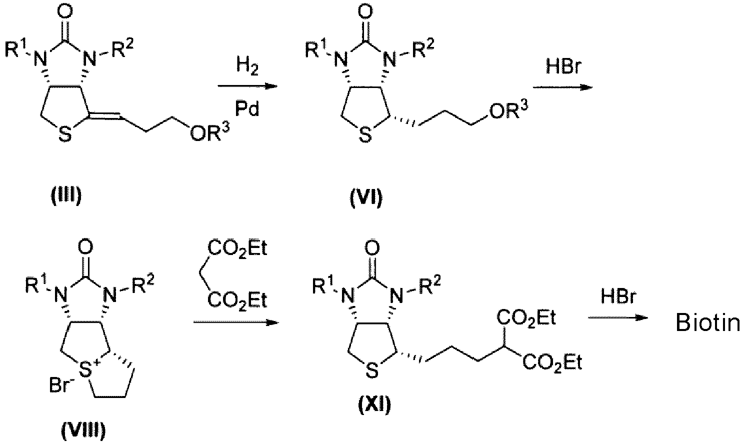

### <Thienoimidazole Derivative Substituted with Straight-Chain Saturated Hydrocarbon Represented by Formula (VI)>

The thienoimidazole derivative substituted with a straight-chain saturated hydrocarbon is represented by the following formula (VI). The thienoimidazole derivative substituted with a straight-chain saturated hydrocarbon may be used as an intermediate for the biotin synthesis as described above.

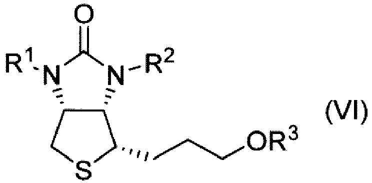

In formula (VI), R¹ and R² are the same as defined in formula (I). R³ is the same as defined in formula (1).

The thienoimidazole derivative substituted with a straight-chain saturated hydrocarbon can be obtained, for example, by contacting the vinyl sulfide derivative represented by formula (III) obtained by the method according to the above-described embodiment with hydrogen (H₂) in the presence of a catalyst.

Examples of the catalyst that may be used include a platinum catalyst such as platinized carbon and platinum oxide; a palladium catalyst such as palladium black, palladium on carbon, palladium acetate, palladium chloride and palladium oxide; a nickel catalyst such as Raney nickel; a cobalt catalyst such as Raney cobalt; a ruthenium catalyst such as ruthenium chloride; an iridium catalyst such as iridium chloride; and an iron catalyst such as iron powder. Preferably, at least one of the Raney nickel and the palladium on carbon is used.

The amount of the catalyst is, for example, 0.001 to 1000 mol%, and preferably 0.1 to 800 mol%, relative to the amount of the vinyl sulfide derivative used as a substrate.

When the vinyl sulfide derivative represented by formula (III) is contacted with hydrogen in the presence of the catalyst, the hydrogen pressure is set to, for example, 1 to 150 atm, and preferably 1 to 50 atm.

When the vinyl sulfide derivative represented by formula (III) is contacted with hydrogen in the presence of the catalyst, the contact temperature is set to, for example, 10 to 200°C, and preferably 25 to 150°C.

When the vinyl sulfide derivative represented by formula (III) is contacted with hydrogen in the presence of the catalyst, the contact time is set to, for example, 0.5 to 100 hours, and preferably 1 to 72 hours.

Contacting the vinyl sulfide derivative represented by formula (III) with hydrogen in the presence of the catalyst may be carried out in the presence of a solvent. Examples of the solvent that may be used include methanol, ethanol, isopropanol, butanol, 2-butanol, ethyleneglycol, 1,2-dimethoxyethane, methyl cellosolve, ethyl acetate, methyl acetate, THF, cyclopentyl methyl ether, 1,4-dioxane, acetic acid, water and a mixed solvent thereof. Preferably, methanol or a mixed solvent of methanol and water is used.

The amount of the solvent used is set to, for example, 1 to 200 mL, and preferably 3 to 100 mL, relative to 1 g of the vinyl sulfide derivative used as a substrate.

### <Method of Producing Hydroxythienoimidazole Derivative Represented by Formula (IV)>

The hydroxythienoimidazole derivative represented by formula (IV) can be produced by the same method as the above-described method of producing the hydroxythienoimidazole derivative represented by formula (II), except that a second Grignard reagent is used instead of the first Grignard reagent.

Specifically, the method of producing the hydroxythienoimidazole derivative represented by formula (IV) includes a step of mixing the thiolactone derivative represented by formula (I), the Grignard reagent represented by formula (2), and the copper salt.

When the thiolactone derivative represented by formula (I) is mixed with the Grignard reagent represented by formula (2) in the presence of the copper salt, an addition reaction proceeds to generate an intermediate represented by the following formula (Ib). Then, it is believed that hydrolysis of this intermediate gives the hydroxythienoimidazole derivative represented by formula (IV). This hydrolysis reaction can be performed by treating the intermediate with water or an acid. As the acid, at least one acid selected from hydrochloric acid, sulfuric acid, phosphoric acid, methanesulfonic acid, para-toluenesulfonic acid, formic acid, acetic acid, propionic acid, ammonium chloride and silica gel can be used.

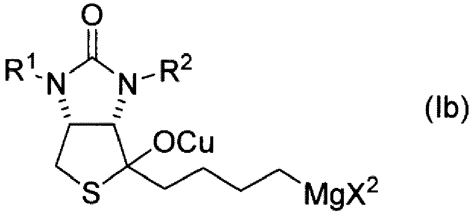

In formula (Ib), R¹ and R² are the same as defined in formula (I). X² is the same as defined in formula (2).

### <Second Grignard Reagent>

The second Grignard reagent is represented by the following formula (2).

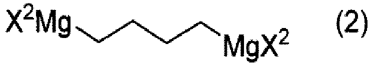

In formula (2), X² is each independently a halogen atom. The halogen atom may be selected from a fluorine atom, a chlorine atom, a bromine atom and an iodine atom. Preferably, the halogen atom is a chlorine atom or a bromine atom. Two X² may be halogen atoms of the same type or halogen atoms of different types.

The second Grignard reagent can be produced, for example, by the same method as the above-described method of producing the first Grignard reagent, except that an organohalogen derivative represented by the following formula (2a) is used instead of the organohalogen derivative represented by formula (1a).

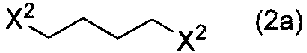

In formula (2a), X² is the same as defined in formula (2).

In the production of the hydroxythienoimidazole derivative represented by formula (IV), it is preferable to use an organocopper reagent obtained by mixing the second Grignard reagent and the copper salt, as is the case with the method of producing a hydroxythienoimidazole derivative represented by formula (II).

In the organocopper reagent, the second Grignard reagent and the copper salt are believed to form a copper complex represented by the following formula (4).

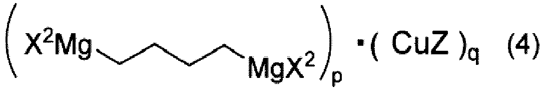

In formula (4), X² is the same as defined in formula (2). Z represents an anion of the copper salt. p and q each represent an integer of 1 to 3.

The amount of the copper salt used is preferably 0.1 to 2 mol, relative to 1 mol of the second Grignard reagent. The amount of the copper salt used is more preferably 0.5 to 1.5 mol, and still more preferably 0.6 to 1.2 mol, relative to 1 mol of the second Grignard reagent.

The amount of the copper salt used is usually 0.1 to 10 mol, preferably 0.5 to 5 mol, and more preferably 0.5 to 2 mol, relative to 1 mol of the thiolactone derivative.

The amount of the second Grignard reagent used is usually 0.5 to 10 mol, preferably 1.0 to 5 mol, and more preferably 1.0 to 2 mol, relative to 1 mol of the thiolactone derivative.

### <Hydroxythienoimidazole Derivative Represented by Formula (IV)>

The hydroxythienoimidazole derivative is represented by the following formula (IV). The hydroxythienoimidazole derivative may be used as an intermediate for the biotin synthesis as described above.

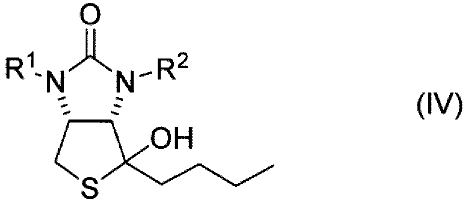

In formula (IV), R¹ and R² are the same as defined in formula (I).

The hydroxythienoimidazole derivative is dehydrated to obtain a n-butylidene sulfide derivative represented by the following formula (V). The n-butylidene sulfide derivative may be used as an intermediate for the biotin synthesis as described above.

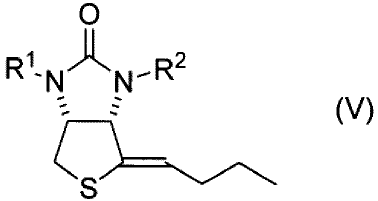

In formula (V), R¹ and R² are the same as defined in formula (I).

Examples of the method of dehydrating the hydroxythienoimidazole derivative include acid treatment and heat treatment. Acid treatment includes, for example, contacting the hydroxythienoimidazole derivative represented by formula (IV) with an acid catalyst. Examples of the acid catalyst include sulfuric acid, hydrochloric acid and a mixture thereof. The temperature for heat treatment is, for example, -20 to 120°C, and preferably 0 to 70°C. Acid treatment and heat treatment may be used in combination.

### EXAMPLES

### <Example 1>

As shown in the following reaction scheme, a compound represented by formula (III') was obtained from a compound represented by formula (I'). In the following formulas, "Bn" represents a benzyl group and "Me" represents a methyl group.

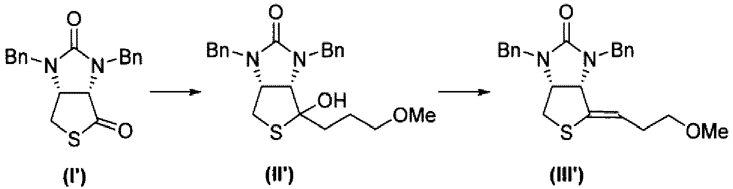

### <Preparation of First Grignard Reagent>

First, a first Grignard reagent was prepared by the following method. THF (1.00 mL) and 1,2-dibromoethane (0.05 mL) were added to Mg (24.3 mg, 1.00 mmol, 2.0 equivalents) to activate Mg. Then, a solution of 1-chloro-3-methoxypropane (54.3 mg, 0.500 mmol, 1.00 equivalent) in THF (1.00 mL) was slowly added dropwise thereto. After all the materials were added, the mixture was stirred at 80°C for 3 hours.

### <Production of Hydroxythienoimidazole Derivative>

The solution of the first Grignard reagent (0.25 M) in THF (1.50 mL, 0.375 mmol, 1.5 equivalents) obtained by the above-described method was added dropwise to a suspension of CuCl (24.8 mg, 0.250 mmol, 1.0 equivalent) in dry THF (1.50 mL) at a temperature of 0°C over a period of 5 minutes, then stirred at a temperature of 0°C for 10 minutes to obtain an organocopper reagent. A solution of the compound represented by formula (I') (84.6 mg, 0.250 mmol, 1.0 equivalent) in THF (2.00 mL) was added dropwise to this organocopper reagent at a temperature of 0°C over a period of 5 minutes, then stirred at a temperature of 0°C over a period of 2 hours to obtain a reaction product. Completion of the reaction was confirmed by subjecting the reaction product to development by thin layer chromatography (TLC). A mixed solvent of ethyl acetate and n-hexane mixed at a volume ratio of 1:1 was used as a developing solvent. The Rf value of the compound represented by formula (II') was 0.11. The plate used for TLC is coated with silica gel. The silica gel acts as an acid, and thus an intermediate (see formula (Ia)) is hydrolyzed to yield a compound represented by formula (II'). 10% H₂SO₄ solution (2 mL) and toluene (5 mL) were added to the reaction product at a temperature of 0°C. Then, the mixture was separated into an aqueous layer and an organic layer by stirring at room temperature over a period of 30 minutes, to obtain the organic layer.

The NMR result for the compound represented by formula (II') was as shown below.
¹H NMR (400 MHz, CDCl₃, 30°C) δ 7.35-7.22 (m, 10H), 5.16-5.10 (m, 1H), 4.86-4.78 (m, 1H), 4.43 (s, 1H), 4.17-3.96 (m, 3H), 3.67 (dd, J=15.7, 9.3 Hz, 1H), 3.61-3.32 (m, 5H), 3.04-2.77 (m, 2H), 2.38-2.31 (m, 1H), 2.01-1.67 (m, 3H).

### <Production of Vinyl Sulfide Derivative>

Next, one drop (catalytic amount) of concentrated sulfuric acid was added to the organic layer and stirred at 60°C over a period of 1 hour to obtain a mixture. This mixture was subjected to development by TLC in the same manner as above. The Rf value of the compound represented by formula (III') was 0.50. The mixture was washed three times with 5 mL of 1 M HCl, further washed with 5 mL of brine, and subsequently dried over Na₂SO₄ to obtain a residue. The residue was analyzed by NMR and was confirmed to contain the compound represented by formula (III'). The yield was 97%. The NMR result for the compound represented by formula (III') was as shown below. ¹H NMR (400 MHz, CDCl₃, 30°C) δ 7.36-7.27 (m, 10H), 5.51 (t, J=7.0 Hz, 1H), 4.93 (d, J=15.6 Hz, 1H), 4.79 (d, J=15.3 Hz, 1H), 4.30 (d, J=7.7 Hz, 1H), 4.23 (d, J=15.3 Hz, 1H), 4.10-4.04 (m, 2H), 3.36 (dt, J=12.9, 2.1 Hz, 2H), 3.32 (s, 3H), 3.00-2.92 (m, 2H), 2.41-2.23 (m, 2H).

### <Example 2>

The compound represented by formula (III') was obtained from the compound represented by formula (I') by the same method as that described in Example 1, except that 0.025 mL of 1,2-dibromoethane, 18.6 mg of CuCl (0.188 mmol, 0.75 equivalents) and 1.10 mL of the solution of the first Grignard reagent in THF (0.275 mmol, 1.1 equivalents) were used. The yield of the compound represented by formula (III') was 100%.

### <Example 3>

As shown in the following reaction scheme, a compound represented by formula (V') was obtained from a compound represented by formula (I'). In the following formulas, "Bn" represents a benzyl group.

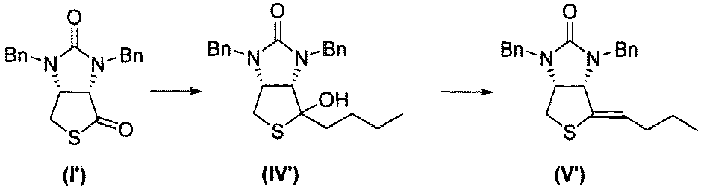

### <Preparation of Second Grignard Reagent>

First, a second Grignard reagent was prepared by the following method. THF (1.00 mL) and 1,2-dibromoethane (0.05 mL, 0.58 mmol) were added to Mg (48.6 mg, 2.00 mmol, 4.0 equivalents) to activate Mg. Then, a solution of 1,4-dichlorobutane (63.5mg, 0.500 mmol, 1.00 equivalent) in THF (1.00 mL) was slowly added dropwise thereto. After all the materials were added, the mixture was stirred at 80°C for 3 hours.

### <Production of Hydroxythienoimidazole Derivative>

The solution of the second Grignard reagent (0.25 M) in THF (1.10 mL, 0.275 mmol, 1.1 equivalents) obtained by the above-described method was added dropwise to a suspension of CuCl (27.2 mg, 0.275 mmol, 1.1 equivalents) in dry THF (2.00 mL) at a temperature of 0°C over a period of 5 minutes, then stirred at a temperature of 0°C for 10 minutes to obtain an organocopper reagent. A solution of the compound represented by formula (I') (84.6 mg, 0.250 mmol, 1.0 equivalent) in THF (2.00 mL) was added dropwise to this organocopper reagent at a temperature of 0°C over a period of 5 minutes, then stirred at a temperature of 0°C over a period of 1 hour to obtain a reaction product. Completion of the reaction was confirmed by subjecting the reaction product to development by thin layer chromatography (TLC). A mixed solvent of ethyl acetate and n-hexane mixed at a volume ratio of 1:1 was used as a developing solvent. The Rf value of the compound represented by formula (IV') was 0.15. The plate used for TLC is coated with silica gel. The silica gel acts as an acid, and thus an intermediate (see formula (Ib)) is hydrolyzed to yield the compound represented by formula (IV'). 10% H₂SO₄ solution (2 mL) and toluene (5 mL) were added to the reaction product at a temperature of 0°C. Then, the mixture was separated into an aqueous layer and an organic layer by stirring at room temperature over a period of 30 minutes to obtain the organic layer.

### <Production of Vinyl Sulfide Derivative>

Next, one drop (a catalytic amount) of concentrated sulfuric acid was added to the organic layer and stirred at 60°C over a period of 1 hour to obtain a mixture. This mixture was subjected to development by TLC in the same manner as above. The Rf value of the compound represented by formula (V') was 0.6. The mixture was washed three times with 5 mL of 1 M HCl, further washed with 5 mL of brine, and subsequently dried over Na₂SO₄ to obtain a residue. The residue was analyzed by NMR and was confirmed to contain the compound represented by formula (V'). The yield was 36%. The NMR result for the compound represented by formula (V') was as shown below. ¹H NMR (400 MHz, CDCl₃, 30°C) δ 7.36-7.24 (m, 10H), 5.46 (t, J=7.2 Hz, 1H), 4.97 (d, J=15.8 Hz, 1H), 4.81 (d, J=15.2 Hz, 1H), 4.31-4.20 (m, 2H), 4.15-4.01 (m, 2H), 3.00-2.92 (m, 2H), 2.04 (hept, J=8.0 Hz, 2H), 1.39 (sext, J=7.3 Hz, 1H), 1.26 (t, J=7.1 Hz, 1H), 0.91 (t, J=7.3 Hz, 3H).

### <Example 4>

As shown in the following reaction scheme, a compound represented by formula (VI') was obtained from a compound represented by formula (III'). In the following formulas, "Bn" represents a benzyl group and "Me" represents a methyl group.

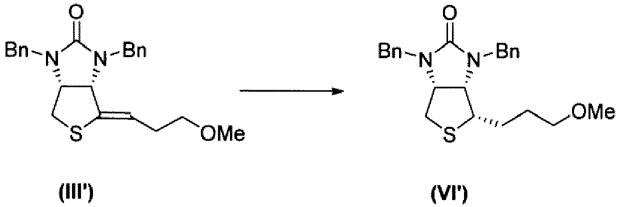

Raney nickel (92.5% by mass (in water), 100 mg, 1.58 mmol, 6.7 equivalents) was quickly weighed out and placed into a glass test tube for autoclaving, and methanol (5.00 mL) was added thereto. A solution of the compound represented by formula (III') (92.6 mg, 0.235 mmol, 1.0 equivalent) in methanol (5.00 mL) was added and stirred at 40°C for 20 hours at a hydrogen pressure of 20 atm. Completion of the reaction was confirmed by subjecting the reaction product to development by thin layer chromatography (TLC). A mixed solvent of ethyl acetate and n-hexane mixed at a volume ratio of 1:1 was used as a developing solvent. The Rf value of the compound represented by formula (VI') was 0.51. After cooling with ice, the mixture was opened to the atmosphere, and the Raney nickel was removed by Celite filtration (ethyl acetate, 5 mL × 4) carefully in such a manner that the Raney nickel does not become dry. The organic layer was washed with 1M aqueous HCl (20 mL × 3) and brine (5 mL × 1), and then dried over Na₂SO₄. The solvent was removed by evaporation to give 90.8 mg of the compound represented by formula (VI') with a yield of 97%. The compound represented by formula (III') remained in only a trace amount. The NMR analysis result for the compound represented by formula (VI') was as shown below.
¹H NMR (400 MHz, CDCl₃, 30°C) δ 7.34-7.24 (m, 10H), 5.10 (d, J=15.2 Hz, 1H), 4.75 (d, J=15.1 Hz, 1H), 4.14 (d, J=15.2 Hz, 1H), 3.99-3.95 (m, 2H), 3.87 (dd, J=9.5, 5.6 Hz, 1H), 3.43-3.35 (m, 2H), 3.34 (s, 3H), 3.335-3.25 (m, 1H), 3.17-3.11 (m, 1H), 2.77-2.66 (m, 1H), 1.90-1.74 (m, 2H), 1.61-1.45 (m, 2H); ¹³C {¹H} NMR (100 MHz, CDCl₃, 30°C) δ 161.1, 137.1, 137.0, 128.8, 128.80, 128.4, 127.8, 72.2, 62.7, 61.3, 58.7, 54.2, 47.9, 46.8, 34.9, 29.2, 25.6; HRMS (FAB⁺) m/z calcd. for C₂₃H₂₉N₂O₂S 397.1950 ([M+H]⁺) found 397.1946.

## Claims

1. A method of producing a hydroxythienoimidazole derivative, comprising
a step of mixing
a thiolactone derivative represented by formula (I): 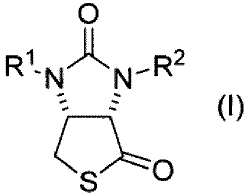
wherein R¹ and R² are each independently an alkyl group, an alkyl group having a substituent, an aryl group, or an aryl group having a substituent,
a Grignard reagent represented by formula (1): 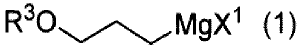
wherein R³ is an alkyl group, an alkyl group having a substituent, an aryl group, or an aryl group having a substituent, and X¹ is a halogen atom, and
a copper salt,
to obtain a hydroxythienoimidazole derivative represented by formula (II): 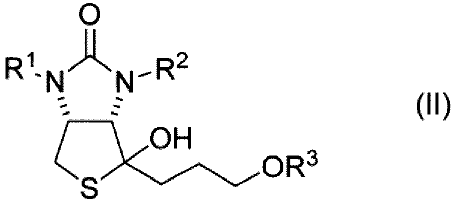
wherein R¹ and R² are the same as defined in formula (I), and R³ is the same as defined in formula (1).

2. The method of producing a hydroxythienoimidazole derivative according to claim 1, wherein, in the step, the Grignard reagent represented by formula (1) and the copper salt are mixed to form an organocopper reagent, and subsequently the organocopper reagent is contacted with the thiolactone derivative represented by formula (I), to obtain the hydroxythienoimidazole derivative represented by formula (II).

3. The method of producing a hydroxythienoimidazole derivative according to claim 1, wherein an amount of the copper salt is 0.05 to 1 mol, relative to 1 mol of the Grignard reagent represented by formula (1).

4. A method of producing a vinyl sulfide derivative, comprising:
a step of obtaining a hydroxythienoimidazole derivative by the method according to any one of claims 1 to 3; and
a step of dehydrating the hydroxythienoimidazole derivative to obtain a vinyl sulfide derivative represented by formula (III): 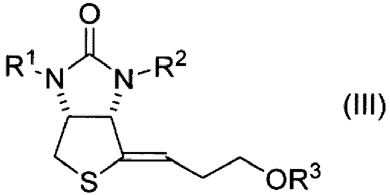
wherein R¹ and R² are the same as defined in formula (I), and R³ is the same as defined in formula (1).

5. A method of producing a thienoimidazole derivative substituted with a straight-chain saturated hydrocarbon, comprising:
a step of obtaining a vinyl sulfide derivative by the method according to claim 4; and
a step of contacting the vinyl sulfide derivative with hydrogen in the presence of a catalyst, to obtain a thienoimidazole derivative substituted with a straight-chain saturated hydrocarbon represented by formula (VI): 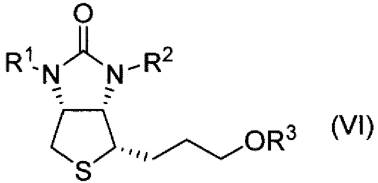
wherein R¹ and R² are the same as defined in formula (I), and R³ is the same as defined in formula (1).

6. A method of producing a hydroxythienoimidazole derivative, comprising
a step of mixing
a thiolactone derivative represented by formula (I): 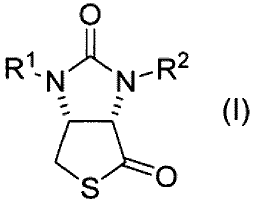
wherein R¹ and R² are each independently an alkyl group, an alkyl group having a substituent, an aryl group, or an aryl group having a substituent,
a Grignard reagent represented by formula (2): 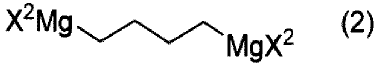
wherein X² is each independently a halogen atom, and
a copper salt,
to obtain a hydroxythienoimidazole derivative represented by formula (IV): 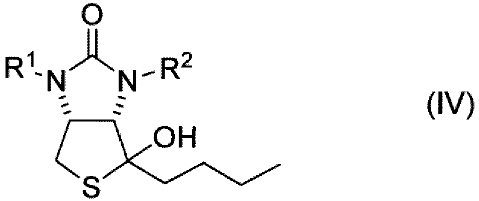
wherein R¹ and R² are the same as defined in formula (I).

7. The method of producing a hydroxythienoimidazole derivative according to claim 6, wherein, in the step, the Grignard reagent represented by formula (2) and the copper salt are mixed to form an organocopper reagent, and subsequently the organocopper reagent is contacted with the thiolactone derivative represented by formula (I), to obtain the hydroxythienoimidazole derivative represented by formula (IV).

8. The method of producing a hydroxythienoimidazole derivative according to claim 6, wherein an amount of the copper salt is 0.1 to 2 mol, relative to 1 mol of the Grignard reagent represented by formula (2).

9. A method of producing an n-butylidene sulfide derivative, comprising:
a step of obtaining a hydroxythienoimidazole derivative by the method according to any one of claims 6 to 8; and
a step of dehydrating the hydroxythienoimidazole derivative to obtain an n-butylidene sulfide derivative represented by formula (V): 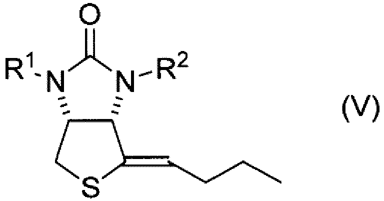
wherein R¹ and R² are the same as defined in formula (I).
